# EUROPÄISCHE PATENTSCHRIFT

(11) **EP 0 443 479 B1**
(45) Veröffentlichungstag und Bekanntmachung des Hinweises auf die Patenterteilung: **13.07.1994**
(21) Anmeldenummer: 91102260.6
(22) Anmeldetag: 18.02.1991
(51) Int. Cl.: G21G 1/00, A61K 43/00

(54) **Verfahren zur Erzeugung von Aktinium-225 und Wismut-213**
Process for producing Actinium-225 and Bismuth-213
Procédé de production d'actinium-225 et de bismuth-213

(30) Priorität: 23.02.1990 LU 87684
(43) Veröffentlichungstag der Anmeldung: 28.08.1991
(73) Patentinhaber: EUROPÄISCHE ATOMGEMEINSCHAFT (EURATOM), L-2920 Luxembourg (LU)
(72) Erfinder: Van Geel, Jacobus N. C., W-7505 Oberweier (DE); Fuger, Jean, W-7519 Wössingen (DE); Koch, Lothar, W-7505 Weingarten (DE)
(74) Vertreter: Weinmiller, Jürgen

(56) Entgegenhaltungen:
- US-A- 2 873 170
- US-A- 4 663 129

## Beschreibung

Die Erfindung bezieht sich auf ein Verfahren zur Erzeugung von Aktinium-225 und Wismut-213.

Radiotherapeutische Methoden zur lokalen Bekämpfung von Krebserkrankungen (Metastasen) gewinnen mit Fortschritten in der Molekularbiologie immer mehr an Bedeutung. Im Prinzip werden dabei kurzlebige alphastrahlende Nuklide in monoklonale Antikörper eingebaut, die, in den Körper des Patienten gebracht, von den malignen Zellen bevorzugt aufgenommen werden und diese durch intensive Bestrahlung mit sehr kurzer Reichweite zerstören. An das Radionuklid werden dabei besondere Anforderungen gestellt: Es muß sich für die Konjugation an einen geeigneten Antikörper binden lassen, muß eine kurze Halbwertzeit haben (in der Größenordnung von einigen Stunden) und seine Zerfallsprodukte müssen chemisch und radiologisch weitgehend unschädlich sein.

Unter den möglichen Kandidaten für ein derartiges Radionuklid spielen Aktinium-225 und Wismut eine hervorragende Rolle, und zwar bezüglich Wismut entweder das Isotop Bi-212 (Halbwertzeit 60,6 Minuten) oder das Isotop Bi-213 (Halbwertzeit 47 Minuten). Die Herstellung von Bi-212 für medizinische Zwecke wurde in der Zeitschrift Int. J. Nucl. Med. Biol. Bend 9 (1982), Seite 83 und in US-A-4663129 beschrieben. Dieses Isotop hat allerdings den Nachteil, daß als Folgeprodukt ein gamma-aktives Thalliumisotop auftritt, das zu einer unerwünschten Strahlenbelastung des Patienten führt.

Dies gilt zwar nicht für Bi-213, jedoch ist dieses Isotop nicht in ausreichenden Mengen verfügbar und kann auch bisher nicht mit vertretbarem Aufwand in diesen Mengen aus U-233 hergestellt werden. Dieses Uranisotop U-233 bildet über mehrere Zerfallsschritte Aktinium-225 und dieses schließlich Bi-213.

Der entscheidende Nachteil dieser Zerfallskette liegt in der geringen Menge Th-229, die beim Zerfall von U-233 entsteht. Da für eine signifikante klinische Versorgung Th-229 in einer Menge zwischen 10 und 100 g benötig wird, müßte U-233 in der Größenordnung von einer Tonne und mit einer langen Lagerzeit von 20 bis 30 Jahren für die Abtrennung zur Verfügung stehen. Derartige Mengen U-233 gibt es aber auf der ganzen Erde nicht.

Aufgabe der Erfindung ist es also, ein Verfahren anzugeben, das mit vernünftigem Aufwand zu einer für die erwähnten radiotherapeutischen Zwecke ausreichenden Menge von Aktinium-225 und Wismut-213 führt.

Das erfindungsgemäße Verfahren besteht darin, daß Radium im thermischen Neutronenfluß eines Kernreaktors bestrahlt wird, daß aus dem Bestrahlungsprodukt der Thoriumanteil chemisch isoliert wird und daß daraus wieder chemisch die durch Zerfall von Thorium 229 laufend gebildeten Radionuklide Radium-225 und Aktinium-225 abgetrennt werden, die dann als Ausgangssubstanz ("Kuh") für die Nuklide Aktinium-225 und Wismut-213 dienen.

Vorzugsweise wird das Radium einem hohen thermischen Neutronenfluß von etwa 5.10¹⁴ /cm².s ausgesetzt.

Die Erfindung wird nun anhand eines Beispiels näher erläutert.

Figur 1 zeigt die zu Wismut-213 führende natürliche Zerfallskette.

Figur 2 zeigt die Bildung von Thorium-229 (in g Th-229) abhängig von der Zeit (in Jahren) bei Bestrahlung von 1 kg Radium-226 in einem thermischen Fluß von 4,7.10¹⁴ Neutronen/cm²s.

Figur 3 zeigt schematisch das ganze erfindungsgemäße Verfahren.

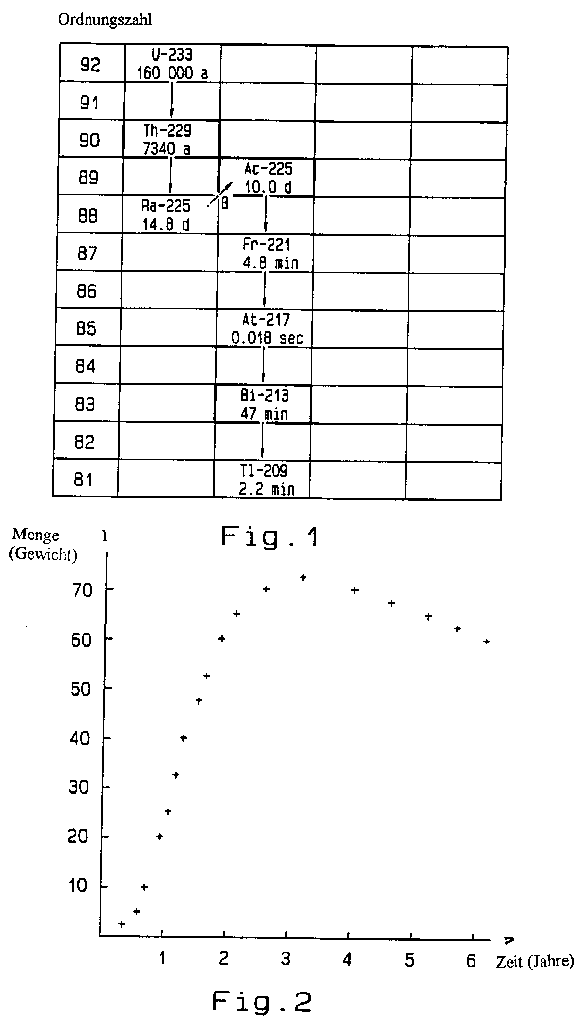

In Figur 1 ist die von Thorium-229 zu Wismut-213 führende Zerfallskette angegeben. Am oberen Ende dieser Zerfallskette ist mit der Ordnungszahl 92 auch das Isotop U-233 angegeben, aus dem durch natürlichen Zerfall das gesuchte Th-229 entsteht, allerdings, wie erwähnt, in unzureichenden Mengen. Der Schlüssel der Herstellung von Ac-225 und Bi-213 in signifikanten Mengen wäre gefunden, wenn das Isotop Th-229 auf andere Weise erzeugt werden könnte als durch Zerfall des U-233.

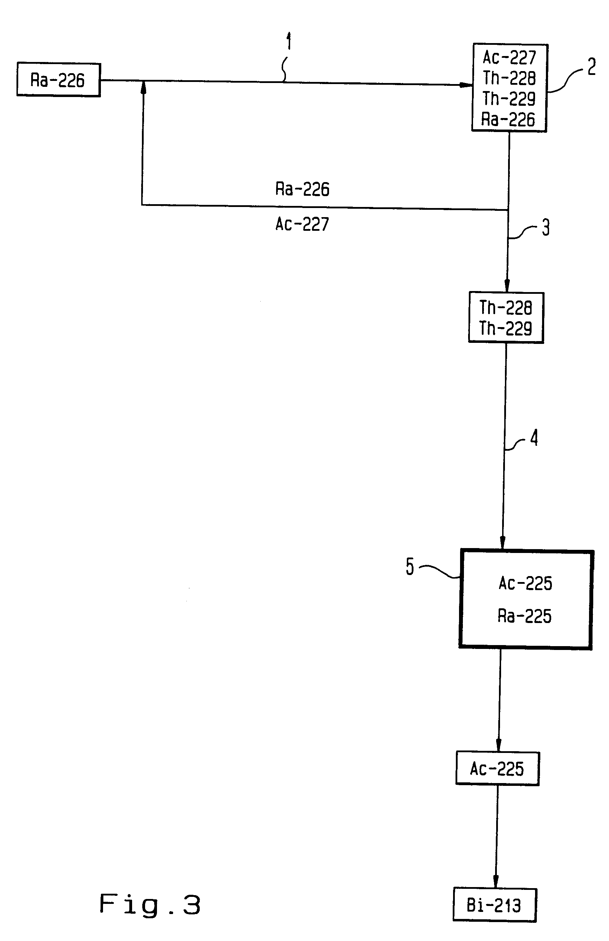

Wie Figur 3 zeigt, dient als Ausgangsprodukt erfindungsgemäß nicht Uran-233, sondern Radium-226 mit der Ordnungszahl 88. Um beispielsweise 100 g Th-229 zu gewinnen, braucht man etwa 1 kg Radium 226. Dieses Ausgangsprodukt wird einige Jahre lang, z.B. 3 Jahre lang, in einem möglichst intensiven thermischen Neutronenfluß von z.B. 4,7.10¹⁴ Neutronen/cm².s eines Hochflußreaktors bestrahlt. Dies wird durch einen Pfeil 1 in Figur 3 angedeutet. Dabei entsteht unter anderem Radium-227, das durch Beta-Zerfall zunächst in Aktinium-227 umgewandelt wird. Durch erneuten Neutroneneinfang entsteht daraus Aktinium-228, das mit einer Halbwertzeit von 6,13 Stunden in Th-228 zerfällt. Unter weiterem Neutronenbeschuß bildet sich hieraus schließlich das gesuchte Isotop Th-229. Durch Rechnung kann man zeigen, daß bei Bestrahlung der angegebenen Menge Radiums mit dem erwähnten Neutronenfluß über einen Zeitraum von 3 Jahren eine Ausbeute an Th-229 in der erwähnten Größenordnung zu erwarten ist (siehe Figur 2).

Das Endprodukt 2 der Bestrahlung besteht also aus einem Gemisch aus Radium-226 und den erwähnten Folgeprodukten Ac-227, Th-228 und Th-229.

Hieraus wird zunächst die Thoriumfraktion chemisch isoliert (Pfeil 3). In dieser bilden sich gemäß dem Zerfallsschema von Figur 1 durch Zerfall von Th-229 die gewünschten Radionuklide Ac-225 und Bi-213.

Es liegt nahe, aus der Thoriumfraktion das Ac-225 zu isolieren und das hierin wachsende Bi-213 seinerseits aus dem Ac-225 abzutrennen. Um aber eine maximale Ausbeute an Ac-225 und Bi-213 zu erreichen, wird nicht nur das Ac-225, sondern auch dessen Mutter Ra-225 (zusammen mit Ra-224, das aus Zerfall von Th-228 entstanden ist) abgetrennt (Pfeil 4). Eine derartige "Kuh" 5 ist auch ein konstanter Lieferant von Ac-225 und Bi-213. Sowohl das Ac-225 als auch das Radium-Isotopengemisch können problemlos versandt und auch im Klinikbetrieb als "Kuh" behandelt werden.

Aus diesem Nuklidengemisch wird das radiotherapeutisch wichtige Aktinium-225 abgetrennt, das gemäß der Zerfallskette in Figur 1 auch für die Erzeugung des radiotherapeutisch wichtigen Bi-213 benutzt werden kann.

## Patentansprüche

1. Verfahren zur Erzeugung von Aktinium-225 und Wismut-213, dadurch gekennzeichnet, daß Radium-226 im thermischen Neutronenfluß eines Kernreaktors bestrahlt wird, daß aus dem Bestrahlungsprodukt der Thoriumanteil chemisch isoliert wird, daß das darin entstehende Radium abgetrennt wird und daß aus dem Radium das einwachsende Aktinium-225 isoliert wird, welches gegebenenfalls als Ausgangsprodukt für Wismut-213 dient.

2. Verfahren nach Anspruch 1, dadurch gekennzeichnet, daß das Radium einem hohen "thermischen" Neutronenfluß von mindestens 4.10¹⁴ Neutronen/cm².s ausgesetzt wird.

## Claims

1. A method for producing actinium-225 and bismuth-213, characterized in that radium-226 is irradiated in the thermal neutron flux of a nuclear reactor, that from the irradiation product the thorium fraction is chemically isolated, that the radium growing therein is separated and that from the radium, the actinium-225 generated therein is isolated, which serves, as the case may be, as basic product for obtaining bismuth-213.

2. A method according to claim 1, characterized in that the radium is submitted to a high thermal neutron flux of at least 4x10¹⁴ neutrons/cm²s.

## Revendications

1. Procédé pour la production d'actinium-225 et de bismuth-213, caractérisé en ce que du radium-226 est irradié dans le flux neutronique thermique d'un réacteur nucléaire, qu'on isole chimiquement du produit de cette irradiation la fraction en thorium, qu'on sépare le radium s'y produisant et que du radium on isole l'actinium-225 qui s'y forme et sert, le cas échéant, comme produit de base pour bismuth-213.

2. Procédé selon la revendication 1, caractérisé en ce que le radium est exposé à un flux "thermique" élevé d'au moins 4.10¹⁴ neutrons/cm²s.
